# EUROPEAN PATENT SPECIFICATION

(11) **EP 2 545 952 B1**
(45) Date of publication and mention of the grant of the patent: **02.04.2014**
(21) Application number: 11174181.5
(22) Date of filing: 15.07.2011
(51) Int. Cl.: A61M 25/00

(54) **Medical device assembly**
Medizinische Vorrichtungsanordnung
Ensemble formant dispositif médical

(43) Date of publication of application: 16.01.2013
(73) Proprietor: Dentsply IH AB, 431 21 Mölndal (SE)
(72) Inventor: Utas, Jan, SE-434 96 KUNGSBACKA (SE); Westman, Robert, SE-791 56 FALUN (SE)
(74) Representative: Lind, Urban Arvid Oskar

(56) References cited:
- WO-A1-03/092779
- GB-A- 1 465 544
- US-A1- 2005 137 582

## Description

### Field of the invention

The present invention relates to a medical device assembly, and in particular a urinary catheter assembly, comprising a separately stored fluid. The invention further relates to a method for producing such a medical device assembly.

### Background of the invention

Many medical devices require to be in contact with a fluid before use. For such medical devices, it is often advantageous to provide a multi-compartment package, wherein the medical device may be stored in one compartment, and the fluid in another compartment, whereby release of the fluid into the compartment housing the medical device can be achieved prior to use, and prior to opening of the package. For example, this is often the case with hydrophilic medical device, wherein the fluid may be a wetting fluid for wetting of the hydrophilic medical device. For example, catheter assemblies are often provided with such separately stored wetting fluids.

Catheters find their use in many different medical applications, such as urinary catheters for bladder drainage. Catheters are normally pre-packed in a package by the manufacturer, in order to maintain the catheter in a clean and preferably sterile condition. Urinary catheters in general need to have a lubricant applied to the outer surfaces thereof to facilitate insertion into the urethra. Especially, for lubrication purposes hydrophilic urinary catheters may have a hydrophilic outer surface coating which should be wetted by a fluid such as water or saline for a certain time period prior to insertion thereof into the urethra of a patient. In order to facilitate the use and to improve cleanliness of the catheter, the assemblies have in recent years developed to comprise a rupturable wetting fluid pouch or container as well.

However, a problem with such assemblies is that they are normally relatively costly to produce. A solution to this problem is presented in US 2005/0043715 by the same applicant. Herein, it is proposed to attach an additional outer layer over the fluid compartment. Hereby, a well sealed and gas impermeable compartment for the fluid may be provided, and at the same time, a cost-efficient material may be chosen for the package. Further, this solution enables a very cost-efficient production. It is further disclosed in said document to provide a weakened joint for separating the compartment housing the catheter and the compartment housing the fluid, to ensure that an induced rupture when applying a pressure on the fluid compartment occurs at the intended location.

However, in this solution, it is still a problem that ruptures may occur at unwanted locations. This will result in spillage of the wetting fluid, which is a great inconvenience for the user. Further, it will also, naturally, lead to insufficient wetting of the catheter.

Accordingly, there is a need for a lean and cost-efficient medical device assembly of the above-discussed type which is easier and more convenient to handle, and with improved rupture control

### Summary of the invention

It is therefore an object of the present invention to alleviate the above-discussed problems. This object is achieved by means of a medical device assembly and a method according to the enclosed claims.
According to the invention there is provided a medical device assembly as claimed in claim 1.

Thus, the package forms two compartments, both forming an integrated part of the package, but being separated from each other, wherein the separation between the compartments provides a rupturable sealed closure.

The additional outer layer, or cover, is provided in order to achieve a stronger and preferably gas impermeable fluid compartment. Due to the use of this additional outer layer, the requirements on the material of the package could be lowered, and the material need e.g. not be gas-impermeable. Hereby, the additional outer layer could provide the impermeability of the compartment wall necessary to alleviate evaporation and maintain the fluid in the compartment during storage. At the same time, only a limited amount of cover material is needed, making the product cost effective to produce.

Further, by attaching the additional outer layer to the package by means of a second joint with a second rupture zone with a strength and/or location that enables opening of the first rupture zone in a state where the package material adjacent to the first rupture zone is unconnected to the additional layer, it has surprisingly been found by the present inventors that a very controllable rupture and fluid release is obtained. When applying a pressure over the fluid compartment, the first rupture zone is free to move in relation to the additional layer, , and thereby, the influence of the additional outer layer of the further rupture is alleviated. Consequently, the first rupture zone will operate according to its intended purpose with greater accuracy, and the risk of rupture at unwanted positions, i.e. at other places than at the intended rupture zone, is greatly diminished.

The strength of the second rupture zone is lower than the strength of the first rupture zone, whereby compression of the fluid within the sealed and closed internal cavity for release of said fluid results in a rupture at the second rupture zone prior to rupture at the first rupture zone. Accordingly, the first and second rupture zone may be overlapping, and the package may initially be connected to the additional layer in the area of the first rupture zone. However, application of a pressure on the fluid compartment will result in opening of the second rupturing zone occurring prior to the opening of the first rupture zone.

By "strength" is here meant the strength to keep the rupture zone closed during application of the compression on the fluid compartment. This is dependent on the actual strength of the joint, but also on the materials of the package and the additional layer, and the force distribution occurring through the package and the additional layer.

According to one line of embodiments, the second rupture zone is separated from the fluid compartment and the first rupture zone in a longitudinal direction of the assembly. Hereby, the package may at all times be unconnected to the additional layer in the area around the first rupture zone, thereby providing the package material in this area sufficient freedom of movement.

Preferably, the additional layer is less elastic, i.e. has a lower elongation at break value, than the material of the package.

The additional outer layer preferably comprises a gas-impermeable material.

The additional cover may also be used as a protection for the fluid against a sterilizing agent used for the sterilization of the catheter and the rest of the catheter assembly. A typical sterilizing agent which could be used for sterilizing the wetting apparatus of the invention is ethylene oxide. Moreover, the fluid in the fluid compartment would normally already be sterile when packed, and need not be further sterilized. Additionally, the sterilizing agent may leave unwanted residual products in the wetting fluid if exposed to the same. For these reasons, it is preferred that the additional cover of the wetting fluid compartment is made of a material which is impermeable or substantially impermeable to ethylene oxide as well as the fluid contained therein. Non-limiting examples of materials satisfying this condition when the fluid is water or saline are aluminium foil laminate, poly(vinylidene chloride) or a laminate comprising metallised film such as metallised poly(ethylene terepthalate), or a silicon oxide coated film, or a laminate comprising aluminum oxide. However, other sterilization methods may also be used, such as sterilization by means of heating or irradiation.

The additional outer layer may be attached to the package by means of an adhesive, welding or any other suitable connection means. However, preferably the additional outer layer is joined to the package by welding, and preferably welding obtained simultaneously with the welding of the joints forming the fluid compartment in the package.

The second joint preferably forms an edge joint extending around the edges of the additional outer layer. Most preferably, the second joint is an edge-joint, leaving an area encircled by the second joint unconnected to the sheet. This ensures that the additional outer layer still serves it protective function as a cover over the fluid compartment, and at the same time ensures a very efficient rupture control.

The package is preferably formed of blanks of a sheet material. Hereby, the package may be formed of two blanks, connected together by means of an edge-joint extending around all the edges, wherein the edge-joints form a compartment or several compartments inside the edge-joints. Thus, each blank preferably provides an inner surface towards the other blank, and another surface towards the ambient atmosphere. The two blanks may be provided by two separate blanks, or by means of a single folded blank, preferably folded along a longitudinal or cross-wise direction.

The fluid compartment forms a sealed and closed internal cavity. Similarly, the storage compartment preferably forms a sealed and closed internal cavity. It is also preferred that the storage compartment houses the entire medical device. However, alternatively, it is possible to enclose only part of the medical device in the storage compartment, whereby, part of the medical device extends out from the package, as is disclosed in the above-discussed US 2005/0043715.

The medical device is preferably a catheter, having on at least part of its surface a hydrophilic surface layer intended to produce a low-friction surface character of the catheter by treatment with a wetting fluid prior to use of the catheter, and wherein the fluid compartment accommodates said wetting fluid. Most preferably, the catheter is a urinary catheter, and preferably a urinary catheter for intermittent use.

In case a hydrophilic urinary catheter is used in the catheter assembly, the fluid is preferably a wetting fluid. Hereby, no additional wetting fluid is needed for activation of the catheter, which entails many advantages. For example, activation of the catheter could easily be accomplished in places where it is normally difficult to find an appropriate wetting fluid for this specific use. Further, it could be ensured that only a sufficiently clean and sterile fluid is used, thereby decreasing the risk for unwanted contamination of the catheter. Still further, the wetting of the catheter may be accomplished in a simpler and more convenient manner.

A suitable fluid is sterile water or a saline solution.

In the assembly of the type discussed above, the fluid in the fluid compartment can be released into the compartment holding the medical device without opening the package. Thus, the fluid can be brought into contact with the medical device, e.g. for wetting a hydrophilic surface on the medical device, while maintaining the sealed and sterile conditions of the package. Release of the fluid is normally performed immediately prior to the intended use of the medical device. However, it is also feasible to release the fluid some time prior to the intended use, such as hours, or even days or weeks prior to the intended use.

The fluid compartment is preferably arranged to keep the wetting fluid separated from at least the insertable part of the catheter during storage and the compartment being openable for activation of the catheter. In such an embodiment, the wetting fluid may be kept separated from the insertable part of the catheter, i.e. the part of the catheter to be inserted through a body opening of the patient, until the time when the catheter is intended to be used. Then, the wetting fluid compartment may be opened by application of a pressure on the fluid compartment.

The additional outer layer is preferably arranged to cover essentially the whole fluid compartment.

The first rupture zone may be formed by means of an adhesive, welding or any other suitable connection means. However, preferably the first rupture zone is be formed by welding.

The package is preferably formed of a flexible plastics material.

Preferably, first and second rupture zones are at least partly overlapping, and most preferably essentially entirely overlapping.

It is further preferred that the package forms an elongate pocket.

Further, the package preferably comprises opening means for opening of the package. The opening means could comprise a peel-off joint, a tear line or the like.

The first and/or second rupture zone(s) may be arranged as a joint provided with at least one point of weakness, in order for an induced rupture to occur in a predetermined position, thereby enabling fluid communication between the compartments. Hereby, the rupture will always occur in the most effective position, e.g. leading to an effective wetting of a catheter surface.

For example, a rupture zone may be formed, in case a welded joint is used, by means of a welding width variation, or a welding strength variation, thus providing the at least one point of weakness. Alternatively, the joint could be arranged in a non-linear arrangement, thus providing the at least one point of weakness. In this case, the joint could be arranged with at least one knee directed towards the wetting fluid compartment. The knee could e.g. have an angled peak portion directed towards the wetting fluid compartment, with an obtuse or acute angle. However, the knee may alternatively have a curved peak portion directed towards the wetting fluid compartment. Hereby, effective rupture control may be achieved, and at the same time a very cost effective and easily producible joint is provided.

According to a second aspect of the invention, there is provided a method for manufacturing a medical device assembly according to claim 12.

According to this method, similar advantages as discussed above are achieved.

Further scope of the applicability of the present invention will become apparent from the detailed description given hereinafter. However, it should be understood that the detailed description and specific examples, while indicating preferred embodiments of the invention, are given by way of illustration only, since various changes and modifications within the scope of the invention will become apparent to those skilled in the art from this detailed description.

### Brief description of the drawings

By way of example embodiments of the invention will now be described with reference to the accompanying drawings in which:
Fig. 1 illustrates an embodiment of a catheter assembly according to the invention, where fig. 1a is a side view of the whole catheter assembly, and fig. 1b is a view of the catheter assembly of fig. 1a illustrating the activation process;
Fig. 2 is a partly broken side view of another embodiment of a catheter assembly according to the invention;
Fig. 3 is a cross-section through the line II-II in fig 2;
Fig. 4 is a partly exploded view of a part of the catheter assembly of Fig. 3;
Fig. 5 illustrates different examples of a weakened joint separating a wetting fluid compartment and a compartment housing the catheter in the package;
Fig. 6a and b illustrate, in a planar top view and a cross-sectional view, respectively, an embodiment of an assembly having first and second rupture zones of different strengths;
Fig. 7 illustrates, in a planar top view, another embodiment of an assembly having first and second rupture zones of different strengths;
Fig. 8 illustrates, in a planar top view, still another embodiment of an assembly having first and second rupture zones of different strengths;
Fig. 9 illustrates, in a planar top view, still another embodiment of an assembly having first and second rupture zones of different strengths; and
Figs. 10 a and b illustrate, in a planar top view and a cross-sectional view, respectively, an embodiment of an assembly having first and second rupture zones being separated in a longitudinal direction of the assembly.

### Description of preferred embodiments

In the following detailed description preferred embodiments of the invention will be described. However, it is to be understood that features of the different embodiments are exchangeable between the embodiments and may be combined in different ways, unless anything else is specifically indicated. In the following detailed exemplary embodiments, the medical device assembly is disclosed as a urinary catheter assembly. However, it is to be acknowledged by any one skilled in the art that the same package arrangement may also be used for other types of medical devices.

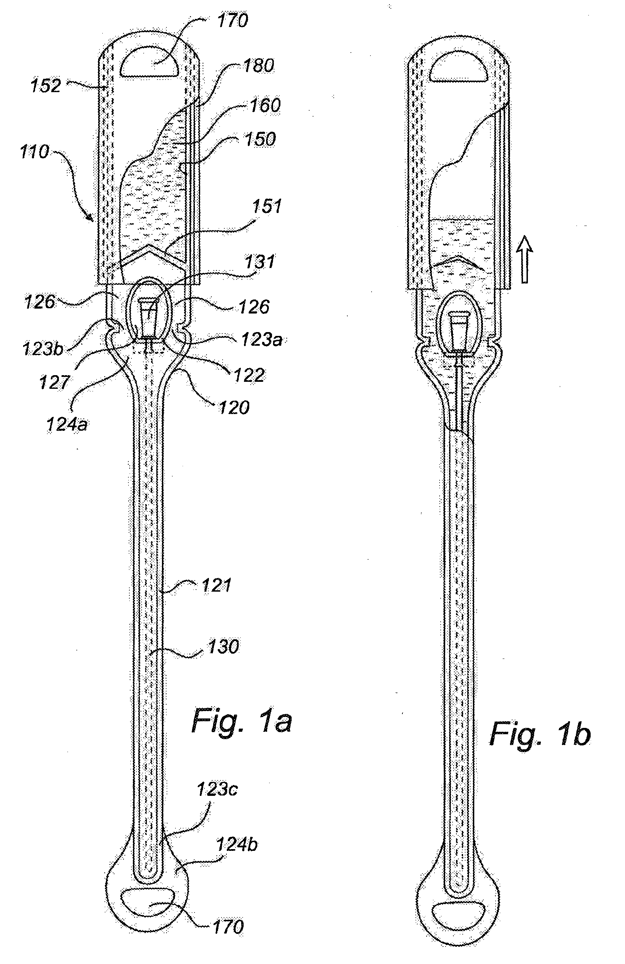

With reference to fig. 1, a first embodiment of the catheter assembly will now be discussed. In this embodiment, the catheter assembly 110 comprises a package 120, preferably of a transparent flexible plastics material. The package 120 has a downwardly extending elongate pocket 121 at the forward end and an opening part 122 with an opening.

The catheter assembly further comprises a catheter, and preferably a hydrophilic urinary catheter 130, with a connection interface for connection to other devices, such as a urine collection bag a drainage tube or the like. In the example illustrated in Fig. 1, the catheter is arranged partly within the package, and partly extending out from the package. Such arrangements are per se disclosed in US 2005/0043715.

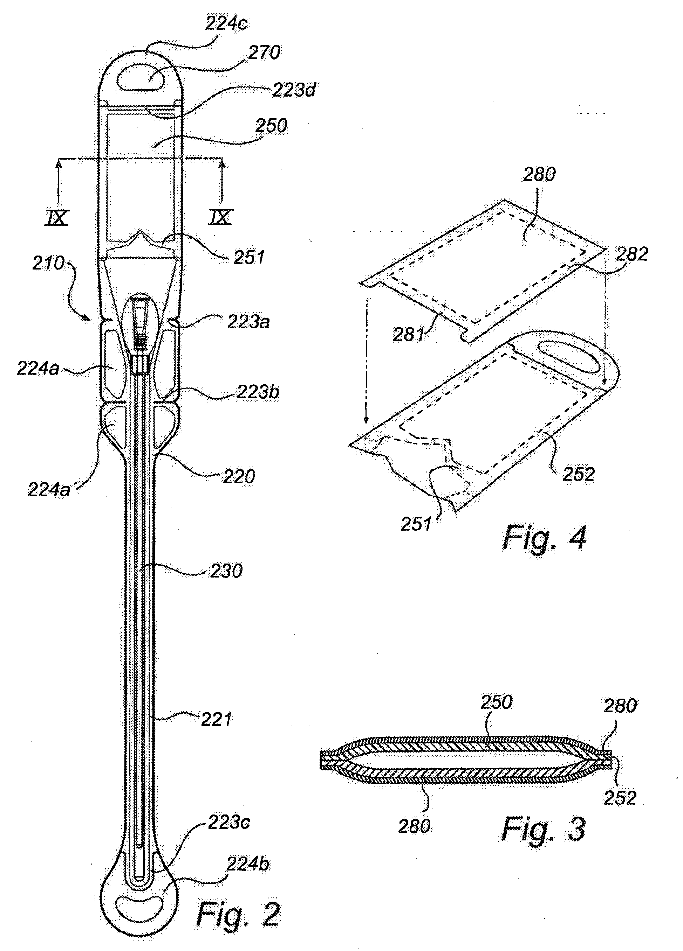

Alternatively, as will be discussed more thoroughly in relation to Fig. 2, the whole catheter may be arranged within the package.

In the embodiment disclosed in Fig. 1, the catheter package 120 is adapted for accommodation of the catheter tube in the elongate pocket 121, and the opening of the opening part 122 is connected to and closed by the connector 131 or rearward end of the catheter. Hereby, the package encloses at least the insertable length of the catheter, but leaves at least part of the catheter outside the package, said part comprising the connection interface.

The assembly also comprises a wetting fluid compartment 150 containing a wetting fluid 160. The wetting fluid compartment is formed in a compartment of the package being separated from the compartment accommodating the catheter. The wetting fluid compartment 150 is in this embodiment arranged in a part of the package extending rearwardly from the catheter, i. e. behind the connector part of the catheter. Said rearward part of the package is preferably in fluid communication with the forward part housing the catheter. This fluid communication may be provided by the arrangement of at least one channel 126 past the catheter connector. Preferably, two such channels 126 are arranged, one on each side of the connector. Thereby the package frames an opening 127 in which the protruding part of the catheter is situated.

The wetting fluid is preferably sterile water or a saline solution.

The wetting fluid compartment of the package is separated from the compartment holding the catheter by means of a rupturable separation joint 151. The separation joint 151 forms a rupture zone between the compartments, e.g. by a weld of less strength than the other welds forming the compartment. Hereby, the wetting fluid may be discharged into the other compartment of the package by compressing the wetting fluid compartment, or by applying a pulling force between the end parts of the assembly.

In order to achieve a stronger and preferably gas impermeable wetting fluid compartment, an additional outer cover 180 is arranged around said compartment.

The package is preferably provided with opening means for facilitating opening of the package in order to expose the catheter for use. The opening means could comprise one or several areas of weakness, such as tear lines 123a, 123b connected to one or several gripping handles 124a, such as a pulling tab. Said opening means could be used to facilitate the removal of the catheter from the package and the insertion into the urethra of the patient

Further, alternatively or additionally, opening means may be arranged close to the distal end of the catheter. Said opening means may comprise a peelable joint 123c connected to tabs 124b extending from the edge for enabling opening by peeling the tabs apart, thereby separating the foil walls of the package. Preferably, the package is arranged to allow a significant degree of separation of the foil walls, thereby making it possible to expose an essential part, and preferably the whole, insertable part of the catheter during this opening process. There is therefore no need to directly handle the catheter 130 during the insertion thereof into the urethra, which is an advantage as the outer surface of the catheter 130 will be slippery due to the wetting procedure and therefore difficult to grip, and furthermore because the possibility of contamination of the catheter at this stage is avoided, whereby the cleanness and sterility of the catheter may be maintained.

At least one, and preferably both, of the end parts of the catheter assembly are preferably provided with gripping means, such as openings 170, for facilitating handling of the catheter assembly.

Due to the use of this additional cover, the requirements on the material of the package could be lowered, and the material need e.g. not be gas-impermeable. Hereby, the additional cover could provide the impermeability of the compartment wall necessary to alleviate evaporation and maintain the wetting fluid in the compartment during storage.

The additional cover may also be used as a protection for the wetting fluid against a sterilizing agent used for the sterilization of the catheter and the rest of the catheter assembly. A typical sterilizing agent which could be used for sterilizing the wetting apparatus of the invention is ethylene oxide. Moreover, the fluid in the fluid compartment would normally already be sterile when packed, and need not be further sterilized. Additionally, the sterilizing agent may leave unwanted residual products in the wetting fluid if exposed to the same. For these reasons, it is preferred that the additional cover of the wetting fluid compartment is made of a material which is impermeable or substantially impermeable to ethylene oxide as well as the fluid contained therein. Non-limiting examples of materials satisfying this condition when the fluid is water or saline are aluminium foil laminate, poly(vinylidene chloride) or a laminate comprising metallised film such as metallised poly(ethylene terepthalate), or a silicon oxide coated film, or a laminate comprising aluminum oxide.

Accordingly, in a production method of this catheter assembly, the package is manufactured, and the catheter is arranged in the package. Further, the wetting fluid is introduced into the wetting fluid compartment, and the additional cover is arranged outside said compartment. Then, the assembly could be sterilized, whereby the additional cover serves as a protection for the wetting fluid against the sterilizing agent.

The additional cover may be attached to the compartment by means of an adhesive, welding or any other suitable connection means, as will be discussed in more detail in the following.

The additional cover as discussed above could also be used for other types of catheter assemblies and medical device assemblies, such as assemblies where the catheter is fully enclosed in the package, or where the connector of the catheter is arranged in a separate compartment of the package.

In a method of wetting the catheter according to this embodiment, the user applies e.g. a compressing force to the wetting fluid compartment 150, thereby forcing open the separation joint 151 and discharging the wetting fluid into the catheter compartment, as is illustrated in fig. 1b. Preferably, the wetting fluid compartment contains a sufficient amount of wetting fluid for the insertable length of the catheter to be sufficiently wetted.

After release of the wetting fluid into the catheter compartment the package may be opened, e.g. at the distal end, as is discussed above, for insertion of the catheter.

With reference to fig. 2, another embodiment of the catheter assembly will now be discussed. This embodiment to a large extent resembles the embodiment discussed with reference to fig 1. The most important differences between the embodiments in fig 1 and fig 2 are that the whole catheter is enclosed in the package in the embodiment in fig 1, and that the wetting fluid compartment is configured somewhat differently.

In this embodiment, the catheter assembly 210 comprises a package, such as a wetting receptacle or bag, 220, preferably of a transparent flexible plastics material. The package 220 has a downwardly extending elongate pocket 221 at the forward end.

As in the previously discussed embodiment, the catheter assembly further comprises a catheter, and preferably a hydrophilic urinary catheter 230, with a connection interface for connection to other devices, such as a urine collection bag a drainage tube or the like. The catheter package 220 is adapted for accommodation of the catheter, and at least the catheter tube is accommodated in the elongate pocket 221. Hereby, the package in this embodiment encloses the whole length of the catheter.

The assembly also comprises a wetting fluid compartment 250 containing a wetting fluid (not illustrated). The wetting fluid compartment is formed in a compartment of the package being separated from the compartment accommodating the catheter. The wetting fluid compartment 250 is in this embodiment arranged in a part of the package extending rearwardly from the catheter, i.e. behind the connector part of the catheter. Said rearward part of the package is preferably in fluid communication with the forward part housing the catheter. This fluid communication may be provided by the compartment of the package housing the catheter being rearwardly open towards the separation to the wetting fluid compartment.

The wetting fluid compartment of the package is separated from the compartment holding the catheter by means of a rupturable separation joint 251. The separation joint 251 forms a rupture zone between the compartments, such as is discussed in more detail in the following. Hereby, the wetting fluid may be discharged into the other compartment of the package by compressing the wetting fluid compartment, or by applying a pulling force between the end parts of the assembly.

In order to achieve a stronger and preferably gas impermeable wetting fluid compartment, an additional cover 280 is arranged around said compartment. This additional cover is arranged as an outer cover arranged over the wetting fluid compartment part of the package. Such an additional cover is very advantageous, and this concept has already been discussed in more detail with reference to fig 1.

In this embodiment, two sheets of outer cover material are arranged over the part of the package forming the wetting fluid compartment. Preferably, the outer cover material sheets are dimensioned essentially only to cover the wetting fluid compartment part of the package. However, larger sheets, possibly covering the whole package are conceivable, as well as smaller sheets, possibly only covering a part of the wetting fluid compartment. Further, a folded sheet of cover material may be used as an alternative two the two separate sheets discussed above.

The attachment of the outer cover will be discussed more thoroughly in the following. This discussion is applicable to both the catheter assembly discussed above with reference to Fig. 2, and also to the assembly discussed with reference to Fig. 1, and other similar types of assemblies.

The cover sheets are preferably welded to the package at the end areas of the sheets, as is best visible in the cross-sectional illustration of fig 3. Accordingly, the sheets are preferably welded to the package close to the welds forming the package.

Even in this embodiment the package could comprise opening means for facilitating opening of the package in order to expose the catheter for use. The opening means could comprise one or several areas of weakness, such as tear lines 223a, 223b connected to one or several gripping handles 224a, 224a', such as a pulling tab. Said opening means could be used to facilitate the removal of the catheter from the package and the insertion into the urethra of the patient.

Further, alternatively or additionally, opening means may be arranged close to the distal end of the catheter. Said opening means may comprise a peelable joint 223c connected to tabs 224b extending from the edge for enabling opening by peeling the tabs apart, thereby separating the foil walls of the package. Preferably, the package is arranged to allow a significant degree of separation of the foil walls, thereby making it possible to expose an essential part, and preferably the whole, insertable part of the catheter during this opening process. There is therefore no need to directly handle the catheter 230 during the insertion thereof into the urethra.

At the other end of the package, preferably arranged relatively close to proximal end of the catheter but on the other side of the wetting fluid compartment, further opening means may be arranged, comprising e.g. a peelable joint 223d connected to tabs 224c extending from the edge for enabling opening by peeling the tabs apart, thereby separating the foil walls of the package. Said opening means may be used for the removal of the catheter from the proximal end after the release of the wetting fluid.

At least one, and preferably both, of the end parts of the catheter assembly are preferably provided with gripping means, such as openings 270, for facilitating handling of the catheter assembly.

The method of wetting the catheter according to this embodiment resembles the wetting process discussed with reference to fig 1. After release of the wetting fluid into the catheter compartment the package may be opened, e.g. at the distal end, as is discussed above, for insertion of the catheter.

The packages as discussed above in relation to Figs. 1 and 2 are preferably made of two joined blanks of a sheet material sealed by their peripheral ends, thereby constituting enclosed compartments for the medical device and the fluid, respectively. The two blanks may be provided by two separate blanks or foils or a single blank/foil. In the latter case, the first and the second blanks are preferably defined by folding the single blank and in preferred embodiment, where the longitudinal extension of the package is larger the width, the single blank is preferably folded either along a line extending in the longitudinal direction or in the cross-wise direction. Thus, the two blanks of a sheet material constituting the package may be provided by bending or folding one blank of a sheet material and by welding the edges together, thus forming an envelope for the catheter. The peripheral parts of the blanks not already joined by means of a possible folding are joined together by means of an edge joint 152, 252 extending around the package, and thereby forming the outer boundaries of the enclosed compartments. A rupturable separation joint 151, 251 extends between two parts of the edge joint 152, 252, and thereby separate the interior of the package into a storage compartment and a fluid compartment. The separation joint 151, 251 also forms a first rupture zone. The rupture zone may be formed over the entire separation joint, or extend over only a limited part of the separation joint. The edge joints 152, 252 and the separation joints 151, 251 are preferably provided by means of welding, but alternatively or additionally, other types of joints, such as adhesive joints, may also be used. For example, the blanks of a sheet material may be joined along edges, e.g. by melting or gluing the foils together or the package may be made from an extruded substantially tubular member being closed in both ends.

The additional outer layer is arranged on at least one of the sides of the fluid compartment, i.e. connected to at least one of the blanks forming the package. Preferably, the additional outer layer is arranged on both sides of the fluid compartment. In this case, two blanks of additional outer layer material are preferably used, and the blanks may be provided by two separate blanks or foils or a single blank/foil. In the latter case, the first and the second blanks are preferably defined by folding the single blank.

As illustrated in Fig. 4, the additional outer layer(s) 280 is/are preferably attached to the package by means of an edge joint 282, encircling the periphery of the additional outer layer, but preferably leaving at least a central part of the additional outer layer unconnected to the package. Preferably, the edge joint 282 is arranged overlaying the corresponding edge joint 152, 252 defining the fluid compartment of the package. The edge joint 282 is preferably a welding joint, but may alternatively or additionally be provided by adhesive joints or the like.

The edge joint 282 attaching the additional outer layer(s) to the package also comprises a second rupture zone 281. The second rupture zone is preferably arranged essentially overlaying and at the same position as the first rupture zone.

The strength of the second rupture zone is lower than the strength of the first rupture zone. Hereby, compression of the fluid within the sealed and closed internal cavity for release of said fluid will result first in a rupture at the second rupture zone. Thereafter, the forces exerted on the joint(s) holding the fluid compartment together will be concentrated to the first rupture zone, whereby a controlled opening and release of the fluid is obtained.

The package is preferably formed by a flexible sheet material, and preferably of a transparent flexible plastics material. The sheet material is further preferably at least substantially gas and water impermeable and which is durable to at least moderate external conditions, such as temperature variations and light. The material should at least substantially maintain its properties over a period of up to 12 or more months, e.g. up to 36 month. The package or at least the blanks may therefore preferably be made from silicone or a thermoplastic elastomeric material, other thermoplastic materials, curable elastomeric materials, polyamide resins or elastomers or any mixture thereof, i.e. the group may comprise materials like, PA, PP, PVC, PU, PE, latex, and/or Kraton^{™}. The foil may also be made from laminates of different materials. One layer may e.g. be a layer of aluminium or similar metal for provision of a completely gas-impermeable package. The two blanks of sheet material may also be provided in two different materials.

The additional outer layer may be formed by the same material as the package, or by a different material. In case a different material is used, the additional outer layer may still be chosen from the group of materials discussed above in relation to the package. Preferably, the material of the additional outer layer comprises or is formed of at least one of the materials: poly(vinylidene chloride) (PVDC), aluminium foil laminates and a laminate comprising a metallised film, for example metallised poly(ethylene terepthalate), and a silicon coated film.

The first and second rupture zones may be provided in various ways. For example, it may be provided by using welds/adhesion of less strength and/or width than in other parts of the joints forming the compartment or attaching the additional outer layer to the package, and/or by introducing other types of weakened points/areas. In the following, various alternatives to provide a rupture zone in the separation joint defining the compartments in the package will be discussed. However, similar arrangements may be used to provide the second rupture zone in the joint connecting the additional outer layer to the package.

Thus, the separation joints 151, 251 may be formed by a separable joint between the compartments, such as weld of less strength than the other welds forming the compartment. Consequently, a whole segment of the total weld joint is weakened, viz. the part of the joint facing the catheter compartment. In order to achieve an even better control of the rupture process, the area of weakness could be even narrower. It is preferred that the weakness is maximized in a limited number of discrete points, such as in one, two or three maxima. However, the points of weakness may also be evenly distributed over a limited area. In that case, it is preferred if the area of weakness is distributed over less than 10-20 % of the joint length. However, the area of weakness may also extend over the entire separable joint.

In the example of fig 1 the weakness has a maximum narrowed down to essentially one point of the length of the joint. This is achieved by means of a non-linear geometrical arrangement of the joint. The joint is here arranged with a knee directed towards the wetting fluid compartment. The knee has an angled peak portion directed towards the wetting fluid compartment, with an obtuse angle. The peak portion defines the area of maximal weakness, and consequently the rupture will inevitably commence in this point, leading to a controllable and predictable rupture process.

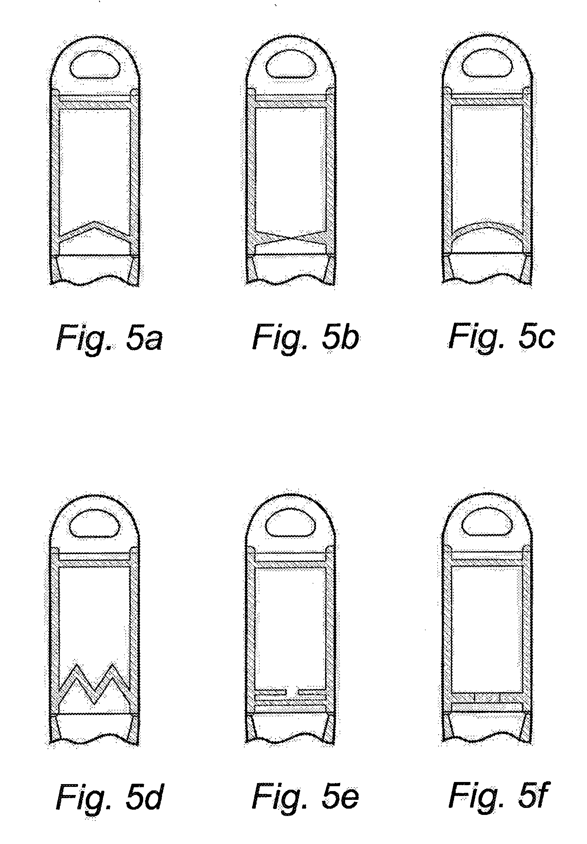

In the embodiment discussed above in relation to fig 2, a similar arrangement is provided. In this embodiment, the rupturable separation joint 251 is of a non-linear geometrical constitution, together with a weld width variation. The joint is here arranged with a knee directed towards the wetting fluid compartment. The knee has an angled peak portion directed towards the wetting fluid compartment, with an acute angle. The peak portion defines the area of maximal weakness, and consequently the rupture will inevitably commence in this point, leading to a controllable and predictable rupture process. Further, this effect is supported and increased by an advantageously arranged width variation of the weld. In this embodiment, the width of the weld is at a minimum at the peak area, and gradually increases towards the ends. Hereby, the strength of the weld is at a minimum at the peak-area, coinciding with the separation force being maximized at the same position due to the geometrical arrangement of the weld. Accordingly, the two parameters, weld width and geometrical arrangement, cooperates to form a very predictable and easily ruptured separation wall. Many different alternatives are conceivable to form the desired rupturable joint between the compartments. Some of these alternatives will now be discussed with reference to fig 5.

In fig 5a, a separation joint is illustrated comprising a knee directed towards the wetting fluid compartment. The knee has an angled peak portion directed towards the wetting fluid compartment, with an obtuse angle. The peak portion defines the area of maximal stress build-up.

In fig 5b, a separation joint is illustrated comprising a width variation of the weld. In this embodiment, the width of the weld is at a minimum essentially at the center of the joint, and gradually increases towards the ends. Hereby, the strength of the weld is at a minimum where the width is the smallest.

In fig 5c, a separation joint is illustrated comprising a knee directed towards the wetting fluid compartment. The knee has a inwardly curved peak portion, directed towards the wetting fluid compartment. The peak portion defines the area of maximal stress build-up.

In fig 5d, a separation joint is illustrated comprising a double knee directed towards the wetting fluid compartment. The joint has two angled peak portion directed towards the wetting fluid compartment, with acute angles. The peak portions defines the area of maximal stress build-up.

In fig 5e, a separation joint is illustrated comprising two welds, wherein a discontinuity is arranged in one of the welds. In this embodiment, an interruption is arranged in the innermost weld, and essentially in the center of the joint. Hereby, the strength of the joint is at a minimum at the discontinuity area.

In fig 5f, a separation joint is illustrated comprising a weld comprising different qualities or weld strengths. In this embodiment, a part of the weld is of a poorer quality and less strength that the rest of the weld, and this part is positioned essentially in the center of the joint. Hereby, the strength of the joint is at a minimum at the area of the weaker weld.

Naturally, other alternatives are conceivable as well. Further, it is also possible to combine two or more of the alternatives, as is e.g. the case with the embodiment discussed in relation to fig 2. Further, the first and second rupture zone may use the same arrangement to obtain weakened points or areas, or use different arrangements.

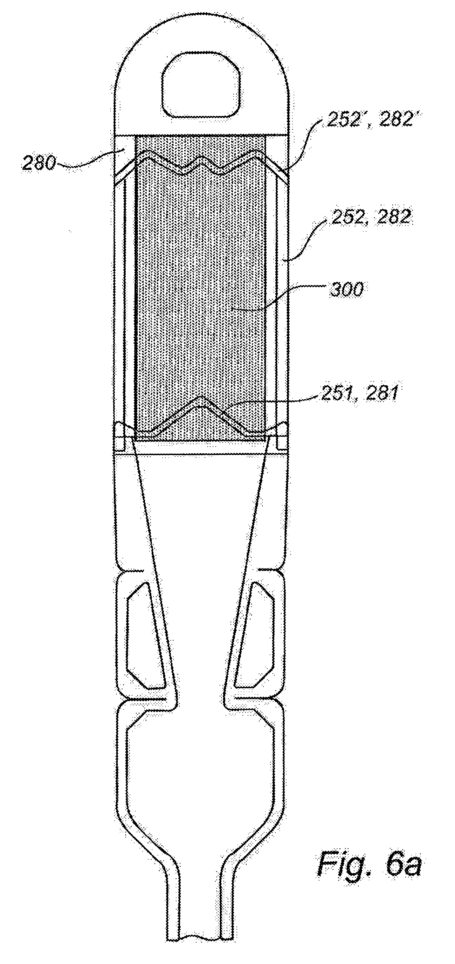

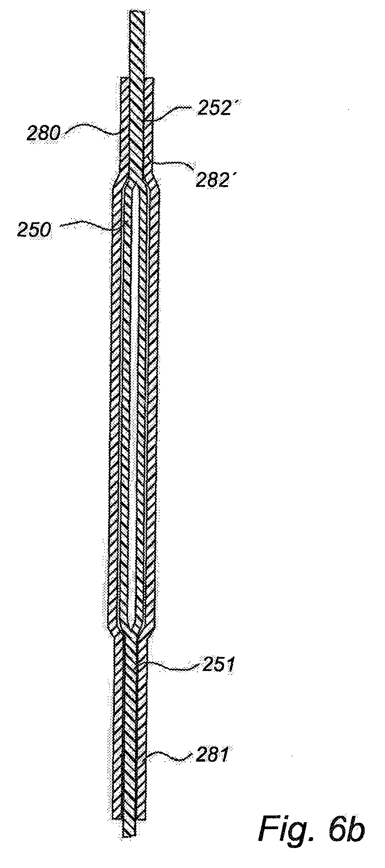

Various ways of obtaining a second rupture zone which has a lower strength and/or which is arranged separated from the first rupture zone will now be discussed in more detail with reference to Figs. 6-10. These embodiments are disclosed in relation to the assembly disclosed in Fig. 2, but naturally, the same principles may be applied also in relation to the assembly of Fig. 1, and other similar types of assemblies.

In Fig. 6, where Fig. 6a is a planar top view, and Fig. 6b is a cross-sectional view in the longitudinal direction of the assembly, a first embodiment providing a second rupture zone with lower strength than the first rupture zone is disclosed. Here, a strength reducing layer 300 is arranged between the foil sheets of the package 250 and the foil of the additional outer layer 280. The strength reducing layer 300 may e.g. be arranged as an innermost layer on the additional outer layer 280. The strength reducing layer 300 can e.g. comprise a hot melt material, such as a hot melt adhesive, a varnish, or the like. The function of the strength reducing layer is to provide a lower strength of the joint when the materials are welded or fused together.

In the embodiment of Fig. 6, the strength reducing layer 300 is arranged to extend over the entire additional outer layer in the longitudinal direction, but only partly over the width of the additional outer layer, leaving the side edges free of the strength reducing layer 300.

In this embodiment, the additional outer layer is attached to the package along joints extending overlapping to the joints forming the fluid compartment, and formed simultaneously with these joints. Thus, the fluid compartment and the attachment of the additional layer are obtained by arranging edge joints 252, 282 along the sides of the package/additional outer layer. These joints are formed outside the strength reducing layer, and are thus not weakened. These joints may e.g. be formed by welding at 125 deg. C. The joints 251, 281, forming the separation between the fluid compartment and the other compartment of the package, extends over the strength reducing layer, and consequently, the strength of the joint 281 is reduced compared to the strength of the joint 251. These joints may e.g. be formed by welding at 97 deg. C. If a hot melt is used for the strength reducing layer, this preferably has a melting temperature of below 78 deg. C. The joints forming the closure of the package, joints 252' and 282', may be performed in a similar way, and e.g. by using welding at 125 deg. C. The joint 282' also occurs over the strength reducing layer, but since this joint is made stronger than joint 281, rupture will occur at joint 281 rather than at joint 282'. There is no connection between the additional outer layer and the package inside the edge joints, as is seen by the illustrative clearance in Fig. 6b.

Since the strength reducing layer extends over the entire additional outer layer in this embodiment, manufacturing of this material becomes very easy. The difference in strength between the joints 282' and 281 can be obtained by using welding at different temperatures, as discussed above, but may additionally or alternatively be obtained in other ways, such as by variation of welding width, etc.

The strength of the welds is preferably in the range 2-5 N/15 mm where the strength reducing layer has been used, and in the range 10-20 N/15 mm in welds without the strength reducing layer.

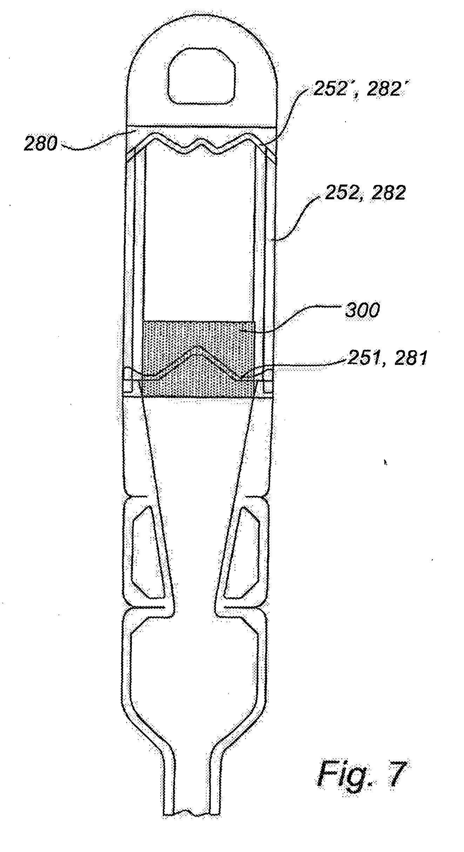

The embodiment of Fig. 7 is similar to the embodiment discussed above in relation to Fig. 6. However, in this embodiment, the strength reducing layer 300 is arranged only over the joint 281. Hereby, no additional measure are required to make the joint 282' stronger than the joint 281.

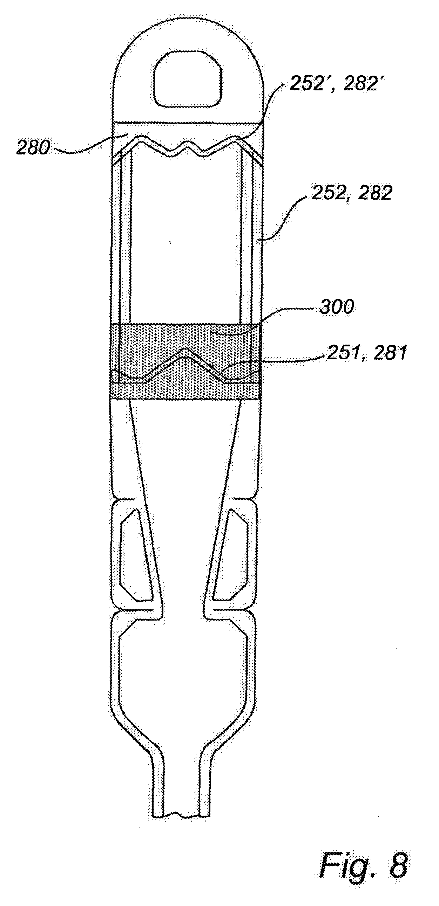

The embodiment of Fig. 8 is similar to the embodiment discussed above in relation to Fig. 7, but here the strength reducing layer 300 is arranged to extend over the entire width of the additional outer layer. Consequently, the second rupture zone may here extend not only over joint 281, but also partly into the side joints 282. Alternatively, the portions of the side joints 282 covered by the strength reducing layer 300 can be made stronger than the joint 281, e.g. by using welding at higher temperature, by using a wider weld seam, etc.

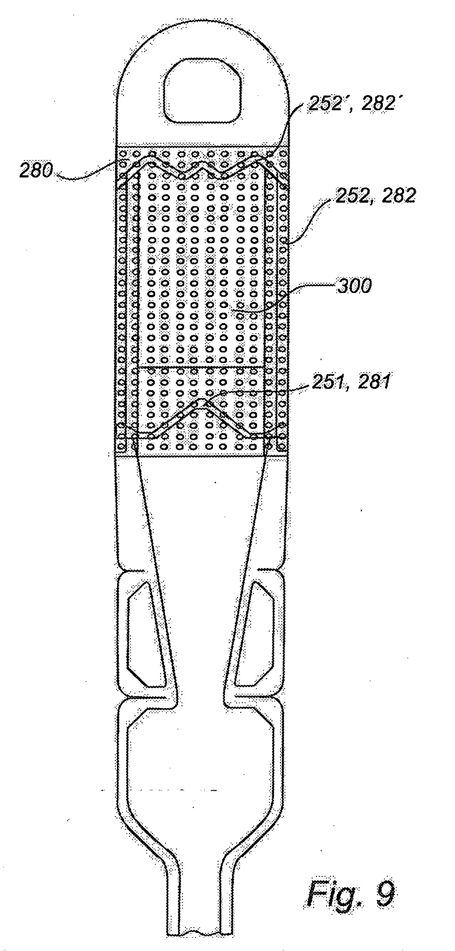

The embodiment of Fig. 9 is similar to the above-discussed embodiments discussed above in relation to Figs. 6-8. Here, the strength reducing layer 300 is arranged over the entire additional outer layer 280, thereby covering all the joints connecting the additional outer layer to the package. It may be ensured that the strength of joint 281 is lower than the strength of the other joints 282, 282' in various ways, as has already been discussed in the foregoing, e.g. by using different welding temperatures.

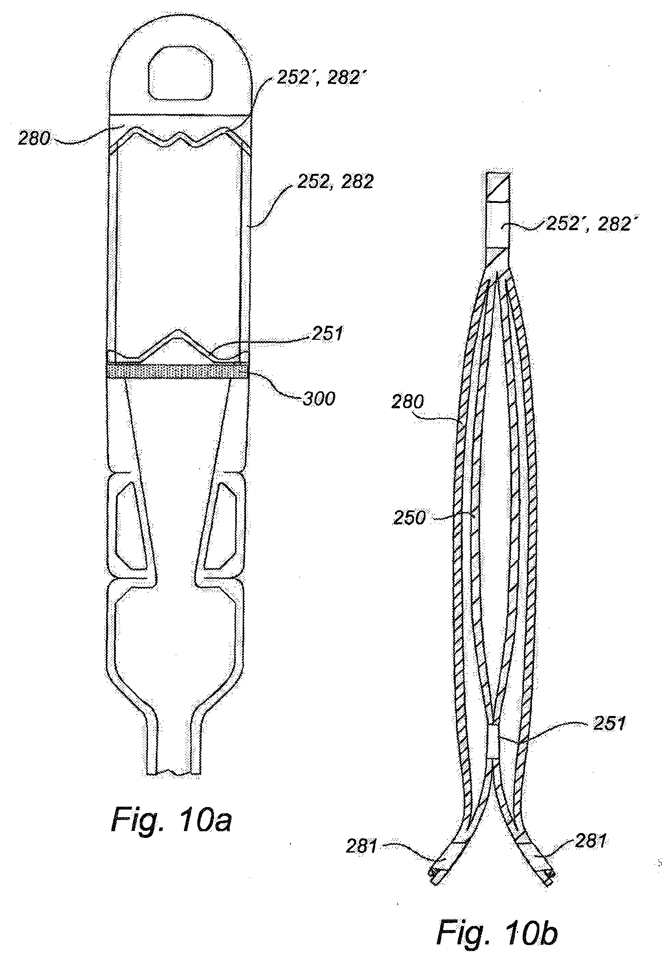

A different line of embodiment is illustrated in Fig. 10, where Fig. 10a is a planar top view, and Fig. 10b is a cross-sectional view in the longitudinal direction of the assembly. Here, the fluid compartment and the attachment of the additional outer layer 280 is obtained as discussed previously, by the arrangement of overlapping joints 252, 252', 282, 282', preferably formed simultaneously, extending along the side edges of the package. However, in this embodiment, the separation joint 251, comprising the first rupture zone, is arranged separated in a longitudinal direction from the joint 281, comprising the second rupture zone. Hereby, the area of the package comprising the joint 251 and the first rupture zone is free to move in relation to the additional outer layer. The joint 281 is preferably arranged over a strength reducing layer 300, here in the form of a hot melt line, and preferably formed by this line, thereby forming and adhesive joint.

In this embodiment, the strength of the second rupture zone in joint 281 is still lower than the strength of the first rupture zone in joint 251.

Production of the different catheter assemblies discussed above is relatively simple. Basically, the production method comprises the steps of providing a package having an opening and a catheter. Thereafter, the catheter is partly introduced into the package, and the package is connected to the catheter, thereby closing said opening, with at least a part of the catheter protruding out from the package. The additional outer layer may be joined to the package after introduction of the catheter and/or after sealing of the package. However, the additional outer layer may also be joined to the package before introduction of the catheter, and may even be attached to the blanks before forming the package.

The invention has now been discussed in relation to different embodiments. However, it should be appreciated by someone skilled in the art that several further alternatives are possible. For example, the features of the different embodiments discussed above could naturally be combined in many other ways. Further, the above discussed assembly structure may be used for other types of medical devices than hydrophilic urinary catheters. For example, it may be used for non-hydrophilic catheters, wherein the fluid would normally be a lubricant. It may also be used to other types of medical devices having hydrophilic surfaces, or where a fluid may be needed for preparation of the medical device for its intended use, such as other types of catheters, such as vascular catheters, cannulas, needles, etc. Similarly, the fluid need not be a wetting fluid, such as water or saline, but may also be fluid lubricants, sterilizing fluids, pharmaceutical fluids, and the like.

Further, the fluid compartment may be arranged in various part of the package, such as in a rearward part, a forward part, or be arranged centrally or laterally. Thus, in case of a catheter assembly, the fluid compartment may be arranged close to the distal part of the catheter, close to the proximal part of the catheter, or in any other suitable location in the assembly.

Preferably the wetting fluid could be discharged without breaking or rupturing the package, even though this may not be necessary, depending on the intended use, etc.

Still further, the means for opening of the package could be any suitable opening means, such as tear lines, peelable joints, breakable areas of weakness, detachable or openable caps or closings, and the like.

Many different materials could also be used for the different parts of the catheter assembly.

Ethylene oxide sterilization could be used for sterilization of the catheter assemblies discussed above. However, many other types of sterilization processes could of course be used instead, for example by irradiation in which case the fluid in the compartment could be sterilized in situ at the same time as the rest of the components of the assembly. Steam treatment may also be used for sterilization.

It will be appreciated by those versed in the art that several such alternatives similar to those described above could be used without departing from the invention, and all such modifications should be regarded as a part of the present invention, as defined in the appended claims.

## Claims

1. A medical device assembly comprising:
a medical device;
a fluid (160);
a package (120; 220) forming a storage compartment, which at least partly accommodates the medical device, and a fluid compartment (150; 250), housing the fluid, wherein a first joint (151; 251) separates the storage compartment and the fluid compartment, the first joint forming a first rupture zone; and
an additional outer layer (180; 280) arranged to at least partly cover the fluid compartment, wherein said additional outer layer is joined to the package by a second joint (281, 282, 282'), wherein at least part of the second joint forms a second rupture zone;
wherein the strength and/or location of the second rupture zone is adapted to ensure that compression of the fluid within the sealed and closed internal cavity for release of said fluid results in opening of said first rupture zone in a state where the package at and in the vicinity of the first rupture zone is unconnected to the additional outer layer;
**characterized in that** the strength of the second rupture zone is lower than the strength of the first rupture zone, whereby compression of the fluid (160) within the sealed and closed internal cavity for release of said fluid results in a rupture at the second rupture zone prior to rupture at the first rupture zone.

2. The assembly of claim 1, wherein the second rupture zone is separated from the fluid compartment (150; 250) and the first rupture zone in a longitudinal direction of the assembly.

3. The assembly of any one of the preceding claims, wherein the second joint (281, 282, 282') forms an edge joint extending around the edges of the additional outer layer.

4. The assembly of any one of the preceding claims, wherein the package is formed of blanks of a sheet material.

5. The assembly of any one of the preceding claims, wherein the medical device is a catheter, having on at least part of its surface a hydrophilic surface layer intended to produce a low-friction surface character of the catheter by treatment with a wetting fluid prior to use of the catheter, and wherein the fluid compartment accommodates said wetting fluid.

6. The assembly of claim 5, wherein the catheter is a urinary catheter (130; 230), and preferably a urinary catheter for intermittent use.

7. The assembly of any one of the preceding claims, wherein the additional outer layer (180; 280) is arranged to cover essentially the whole fluid compartment (150; 250).

8. The assembly of any one of the preceding claims, wherein the package is formed of a flexible plastics material.

9. The assembly of any one of the preceding claims, wherein the additional outer layer (180; 280) comprises a gas-impermeable material.

10. The assembly of any one of the preceding claims, wherein the second joint (281, 282, 282') is an edge-joint, leaving an area encircled by the second joint unconnected to the sheet.

11. The assembly of any one of the preceding claims, wherein the first and second rupture zones are at least partly overlapping.

12. A method for manufacturing a medical device assembly comprising:
providing a package (120; 220) forming a storage compartment and a fluid compartment (150; 250);
arranging a medical device in said storage compartment;
arranging a fluid (160) in said fluid compartment (150; 250);
separating said fluid compartment (150; 250) and said storage compartment with a first joint, the first joint forming a first rupture zone; and
arranging an additional outer layer (180; 280) over at least part of the fluid compartment (150; 250), wherein said additional outer layer (180; 280) is joined to the package by a second joint (281, 282, 282'), wherein at least part of the second joint forms a second rupture zone;
wherein the strength and/or location of the second rupture zone is adapted to ensure that compression of the fluid (160) within the sealed and closed internal cavity for release of said fluid results in opening of said first rupture zone in a state where the package at and in the vicinity of the first rupture zone is unconnected to the additional outer layer,
**characterized in that** the strength of the second rupture zone is lower than the strength of the first rupture zone, whereby compression of the fluid (160) within the sealed and closed internal cavity for release of said fluid results in a rupture at the second rupture zone prior to rupture at the first rupture zone.

13. The method of claim 12, wherein the second rupture zone is separated from the fluid compartment and the first rupture zone in a longitudinal direction of the assembly.

## Patentansprüche

1. Medizinische Vorrichtungsanordnung, umfassend:
eine medizinische Vorrichtung;
ein Fluid (160);
eine Verpackung (120; 220), die ein Speicherabteil, das die medizinische Vorrichtung mindestens teilweise aufnimmt, und ein Fluidabteil (150; 250), welches das Fluid unterbringt, bildet, wobei eine erste Dichtung (151; 251) das Speicherabteil und das Fluidabteil trennt, wobei die erste Dichtung eine erste Bruchzone bildet; und
eine zusätzliche Außenschicht (180; 280), die angeordnet ist, um das Fluidabteil mindestens teilweise abzudecken, wobei die zusätzliche Außenschicht mit der Verpackung durch eine zweite Dichtung (281, 282, 282') verbunden ist, wobei mindestens ein Teil der zweiten Dichtung eine zweite Bruchzone bildet;
wobei die Festigkeit und/oder Position der zweiten Bruchzone geeignet ist bzw. sind, um sicherzustellen, dass die Kompression des Fluids im Innern des abgedichteten und geschlossenen internen Hohlraums zur Freigabe des Fluids zum Öffnen der ersten Bruchzone in einem Zustand führt, bei dem die Verpackung an oder in der Nähe von der ersten Bruchzone nicht mit der zusätzlichen Außenschicht verbunden ist;
**dadurch gekennzeichnet, dass** die Festigkeit der zweiten Bruchzone geringer als die Festigkeit der ersten Bruchzone ist, wodurch die Kompression des Fluids (160) im Innern des abgedichteten und geschlossenen internen Hohlraums zur Freigabe des Fluids zu einem Bruch an der zweiten Bruchzone vor einem Bruch an der ersten Bruchzone führt.

2. Anordnung nach Anspruch 1, wobei die zweite Bruchzone von dem Fluidabteil (150; 250) und der ersten Bruchzone in einer Längsrichtung der Anordnung getrennt ist.

3. Anordnung nach einem der vorhergehenden Ansprüche, wobei die zweite Dichtung (281, 282, 282') eine Randdichtung bildet, die sich um die Ränder der zusätzlichen Außenschicht erstreckt.

4. Anordnung nach einem der vorhergehenden Ansprüche, wobei die Verpackung aus Zuschnitten von Plattenmaterial gebildet ist.

5. Anordnung nach einem der vorhergehenden Ansprüche, wobei die medizinische Vorrichtung ein Katheter ist, der auf mindestens einem Teil seiner Oberfläche eine hydrophile Oberflächenschicht aufweist, die dazu gedacht ist, durch eine Behandlung mit einem Benetzungsfluid vor der Verwendung des Katheters eine reibungsarme Beschaffenheit des Katheters zu erzeugen, und wobei das Fluidabteil das Benetzungsfluid aufnimmt.

6. Anordnung nach Anspruch 5, wobei der Katheter ein Harnkatheter (130; 230) und bevorzugt ein Harnkatheter zur zeitweiligen Verwendung ist.

7. Anordnung nach einem der vorhergehenden Ansprüche, wobei die zusätzliche Außenschicht (180; 280) angeordnet ist, um im Wesentlichen das gesamte Fluidabteil (150; 250) abzudecken.

8. Anordnung nach einem der vorhergehenden Ansprüche, wobei die Verpackung aus einem flexiblen Plastikmaterial gebildet ist.

9. Anordnung nach einem der vorhergehenden Ansprüche, wobei die zusätzliche Außenschicht (180; 280) ein gasundurchlässiges Material umfasst.

10. Anordnung nach einem der vorhergehenden Ansprüche, wobei die zweite Dichtung (281, 282, 282') eine Randdichtung ist, die einen Bereich überlässt, der von der zweiten Dichtung umgeben ist, die nicht mit der Platte verbunden ist.

11. Anordnung nach einem der vorhergehenden Ansprüche, wobei sich die ersten und zweiten Bruchzonen mindestens teilweise überlappen.

12. Verfahren zum Herstellen einer medizinischen Vorrichtungsanordnung, umfassend folgende Schritte:
Bereitstellen einer Verpackung (120; 220), die ein Speicherabteil und ein Fluidabteil (150; 250) bildet;
Anordnen einer medizinischen Vorrichtung in dem Speicherabteil;
Anordnen eines Fluids (160) in dem Fluidabteil (150; 250);
Trennen des Fluidabteils (150; 250) und des Speicherabteils mit einer ersten Dichtung, wobei die erste Dichtung eine erste Bruchzone bildet; und
Anordnen einer zusätzlichen Außenschicht (180; 280) über mindestens einem Teil des Fluidabteils (150; 250), wobei die zusätzliche Außenschicht (180; 280) mit der Verpackung durch eine zweite Dichtung (281, 282, 282') zusammengefügt ist, wobei mindestens ein Teil der zweiten Dichtung eine zweite Bruchzone bildet;
wobei die Festigkeit und/oder Position der zweiten Bruchzone geeignet ist bzw. sind, um sicherzustellen, dass die Kompression des Fluids (160) in dem abgedichteten und geschlossenen internen Hohlraum zur Freigabe des Fluids zum Öffnen der ersten Bruchzone in einem Zustand führt, in dem die Verpackung an oder in der Nähe von der ersten Bruchzone nicht mit der zusätzlichen Außenschicht verbunden ist,
**dadurch gekennzeichnet, dass** die Festigkeit der zweiten Bruchzone geringer als die Festigkeit der ersten Bruchzone ist, wodurch die Kompression des Fluids (160) im Innern des abgedichteten und geschlossenen internen Hohlraums zur Freigabe des Fluids zu einem Bruch an der zweiten Bruchzone vor einem Bruch an der ersten Bruchzone führt.

13. Verfahren nach Anspruch 12, wobei die zweite Bruchzone von dem Fluidabteil und der ersten Bruchzone in einer Längsrichtung der Anordnung getrennt ist.

## Revendications

1. Assemblage de dispositif médical comprenant:
un dispositif médical;
un fluide (160);
un paquet (120; 220) formant un compartiment de stockage, qui accueille au moins de façon partielle le dispositif médical, et un compartiment à fluide (150; 250), abritant le fluide, dans lequel une première jointure (151; 251) sépare le compartiment de stockage et le compartiment à fluide, la première jointure formant une première zone de rupture; et
une couche extérieure supplémentaire (180; 280) disposée pour couvrir au moins de façon partielle le compartiment à fluide, dans lequel ladite couche extérieure supplémentaire est reliée au paquet par une seconde jointure (281, 282, 282'), dans lequel au moins une partie de la seconde jointure forme une seconde zone de rupture;
dans lequel la force et/ou l'emplacement de la seconde zone de rupture est adaptée pour s'assurer que la compression du fluide au sein de la cavité interne scellée et fermée pour la libération dudit fluide résulte en l'ouverture de ladite première zone de rupture dans un état où le paquet se trouvant à, et au voisinage de, la première zone de rupture n'est pas relié à la couche extérieure supplémentaire;
**caractérisé en ce que** la force de la seconde zone de rupture est inférieure à la force de la première zone de rupture, ainsi la compression du fluide (160) au sein de la cavité interne scellée et fermée pour la libération dudit fluide résulte en une rupture à la seconde zone de rupture prioritairement à une rupture à la première zone de rupture.

2. Assemblage de la revendication 1, dans lequel la seconde zone de rupture est séparée du compartiment à fluide (150; 250) et de la première zone de rupture dans une direction longitudinale de l'assemblage.

3. Assemblage de l'une quelconque des revendications précédentes, dans lequel la seconde jointure (281, 282, 282') forme une jointure de bordure s'étendant autour des bords de la couche extérieure supplémentaire.

4. Assemblage de l'une quelconque des revendications précédentes, dans lequel le paquet est formé d'ébauches d'un matériau en feuilles.

5. Assemblage de l'une quelconque des revendications précédentes, dans lequel le dispositif médical est un cathéter, ayant au moins sur une partie de sa surface, une couche de surface hydrophile prévue pour créer au niveau du cathéter une surface à faible frottement par traitement avec un fluide de mouillage avant utilisation du cathéter, et dans lequel le compartiment à fluide accueille ledit fluide de mouillage.

6. Assemblage de la revendication 5, dans lequel le cathéter est un cathéter urinaire (130; 230), et de préférence un cathéter urinaire destiné à une utilisation intermittente.

7. Assemblage de l'une quelconque des revendications précédentes, dans lequel la couche extérieure supplémentaire (180; 280) est disposée pour couvrir essentiellement tout le compartiment à fluide (150; 250).

8. Assemblage de l'une quelconque des revendications précédentes, dans lequel le paquet est formé de matériaux plastiques flexibles.

9. Assemblage de l'une quelconque des revendications précédentes, dans lequel la couche extérieure supplémentaire (180; 280) comprend un matériau imperméable au gaz.

10. Assemblage de l'une quelconque des revendications précédentes, dans lequel la seconde jointure (281, 282, 282') est une jointure de bordure laissant une surface encerclée par la seconde jointure non reliée à la feuille.

11. Assemblage de l'une quelconque des revendications précédentes, dans lequel la première et la seconde zones de rupture se chevauchent au moins de façon partielle.

12. Procédé pour la fabrication d'un assemblage de dispositif médical comprenant:
la fourniture d'un paquet (120; 220) formant un compartiment de stockage et un compartiment à fluide (150; 250);
la disposition d'un dispositif médical, dans ledit compartiment de stockage;
la disposition d'un fluide(160) dans ledit compartiment à fluide (150; 250);
la séparation dudit compartiment à fluide (150; 250) et dudit compartiment de stockage avec la première jointure, la première jointure formant une première zone de rupture; et
la disposition d'une couche extérieure supplémentaire (180; 280) au moins sur une partie du compartiment à fluide (150; 250), dans lequel ladite couche extérieure supplémentaire (180; 280) est jointe au paquet par une seconde jointure (281, 282, 282'), dans lequel au moins une partie de la seconde jointure forme une seconde zone de rupture;
dans lequel la force et/ou l'emplacement de la seconde zone de rupture est adaptée pour s'assurer que la compression du fluide (160) au sein de la cavité interne scellée et fermée pour la libération dudit fluide résulte en l'ouverture de ladite première zone de rupture dans un état où le paquet se trouvant à, et au voisinage de, la première zone de rupture n'est pas relié à la couche extérieure supplémentaire,
**caractérisé en ce que** la force de la seconde zone de rupture est inférieure à la force de la première zone de rupture, ainsi la compression du fluide (160) au sein de la cavité interne scellée et fermée pour la libération dudit fluide résulte en une rupture à la seconde zone de rupture prioritairement à une rupture à la première zone de rupture.

13. Procédé de la revendication 12, dans lequel la seconde zone de rupture est séparée du compartiment à fluide et de la première zone de rupture dans une direction longitudinale de l'assemblage.
